# FASCICULE DE BREVET EUROPEEN

(11) **EP 1 289 451 B1**
(45) Date de publication et mention de la délivrance du brevet: **01.12.2004**
(21) Numéro de dépôt: 01945426.3
(22) Date de dépôt: 14.06.2001
(51) Int. Cl.: A61F 2/14

(54) **CORNEE SYNTHETIQUE**
KÜNSTLICHE HORNHAUT
PROSTHETIC CORNEA

(30) Priorité: 16.06.2000 FR 0007684; 20.07.2000 FR 0009550
(43) Date de publication de la demande: 12.03.2003
(73) Titulaire: CORNEAL INDUSTRIE, 74370 Pringy (FR)
(72) Inventeur: LACOMBE, Emmanuel, F-75016 PARIS (FR); PAREL, Jean-Marie, MIAMI SHORES, FL 33138 (US); DUCHESNE, Bernard, B-4000 LIEGE (BE); VILLAIN, Franck, F-75020 PARIS (FR)
(74) Mandataire: Dronne, Guy
(86) Numéro de dépôt international: PCT/FR2001/001845
(87) Numéro de publication internationale: WO 2001/095836

(56) Documents cités:
- EP-A- 0 420 549
- WO-A-97/27824
- DE-A- 19 508 922
- US-A- 4 563 779
- US-A- 4 842 599
- US-A- 5 300 116
- US-A- 5 489 301
- US-A- 5 945 498

## Description

La présente invention a pour objet une cornée synthétique.

De façon plus précise, l'invention se rapporte à une prothèse optique qui peut être mise en place dans la cornée après la réalisation d'une ouverture centrale pratiquée dans celle-ci lorsque la cornée ne remplit plus sa fonction de transparence.

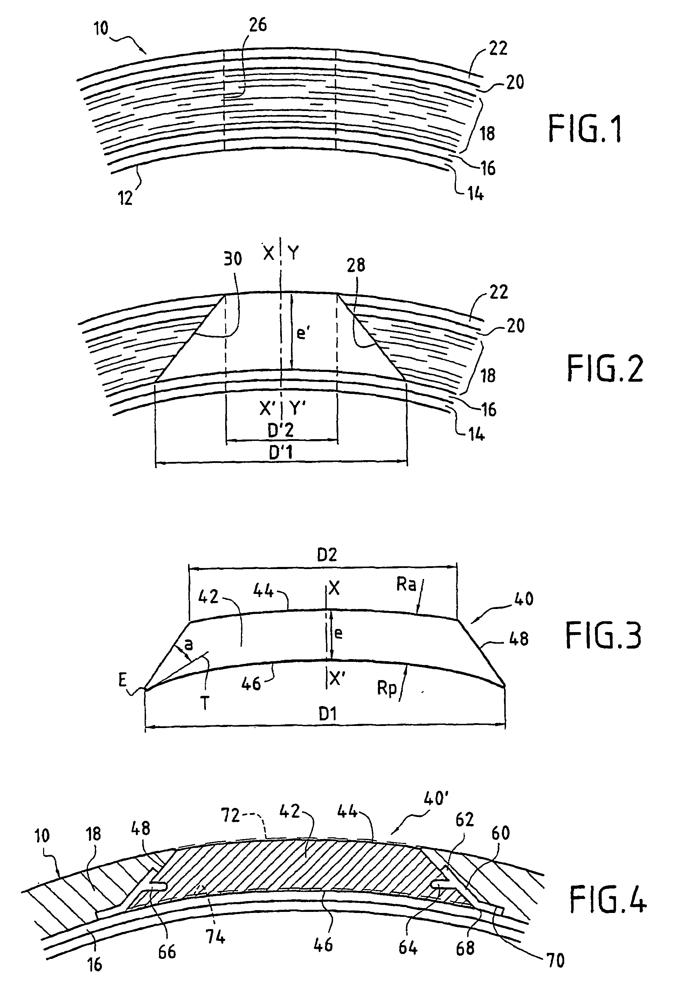

Sur la figure 1 annexée, on a représenté la partie centrale de la cornée 10 d'un oeil. Celle-ci est constituée par une pluralité de membranes ou lamelles disposées les unes sur les autres. A partir de la face postérieure, c'est-à-dire de la face interne 12 de la cornée, on peut distinguer l'endothélium 14, qui est constitué par une couche de cellules qui ne se régénèrent pas, la membrane de Descemet 16, des lamelles intermédiaires 18 formant le stroma de la cornée puis la membrane de Bowman 20 et enfin l'épithélium 22 qui constitue la face antérieure 24 de la cornée. L'épithélium est constitué par plusieurs couches de cellules qui ont une grande propension à la régénération. L'épaisseur totale de la cornée, dans sa région centrale, est de l'ordre de 500 microns et le stroma et l'épithélium représentent à eux deux plus de 97% de l'épaisseur totale.

Dans les techniques connues de mise en place de kératoprothèse, on procède à la réalisation d'une incision en forme de logement cylindrique 26 qui traverse de part en part l'épaisseur de la cornée. Dans le type de kératoprothèse le plus connu dite kératoprothèse intrastromale, la prothèse est constituée par une pièce optique cylindrique disposée dans l'évidement 26 et elle comporte une jupe annulaire qui est insérée entre des lamelles du stroma de la cornée 18 pour assurer le maintien mécanique de la kératoprothèse dans la cornée. Cette technique de kératoprothèse interstromale est connue pour donner des résultats médiocres ou carrément mauvais.

C'est pourquoi, on a proposé notamment, dans la demande de brevet WO 97/27824 au nom de la demanderesse, une autre forme de kératoprothèse dans laquelle la partie optique cylindrique est munie d'une jupe latérale postérieure qui est appliquée contre la face interne de la cornée afin d'assurer la tenue mécanique de la kératoprothèse dans la cornée. Cette solution donne des résultats beaucoup plus satisfaisants.

Quel que soit le type de maintien de la kératoprothèse dans la comée, les techniques antérieures nécessitent la réalisation d'un évidement dans la partie centrale de la comée qui traverse celle-ci de part en part. Le fait de réaliser cette ouverture complète présente un certain nombre d'inconvénients notamment en ce qui concerne les risques de fuites ou d'infections.

Lorsque la cornée est altérée de manière sévère (brûlures, etc.), les kératoprothèses avec appui post-coméen constituent la solution la mieux adaptée.

Dans des cas plus nombreux, l'altération de la cornée est réduite et la membrane de Descemet n'est pas affectée. Une greffe de cornée est alors possible. Cependant, les problèmes liés à la contamination virale et à la pénurie de greffons fait qu'il serait intéressant de disposer d'une cornée synthétique qui puisse être mise en place dans la cornée tout en permettant de conserver la membrane de Descemet et la couche endothéliale afin d'éviter les complications mentionnées ci-dessus dues à une ouverture transfixante dans la cornée.

Le document US 4,842 599 décrit une comée synthétique qui peut être mise en place dans la cornée en conservant la membrane de Descemet. Cependant, pour assurer le maintien mécanique de la cornée synthétique, celle-ci est munie d'une jupe annulaire de fixation, cette jupe comporte des orifices pour la réalisation de sutures. Une telle solution n'est pas satisfaisante car elle entraîne un traumatisme important sur une zone étendue de la cornée.

Le document EP0420549A décrit un implant optique de cornée selon le préambule de la revendication 1.

Un objet de la présente invention est de fournir une cornée synthétique qui puisse être mise en place dans la cornée et maintenue dans celle-ci de façon efficace tout en évitant d'avoir à réaliser une perforation complète de la cornée, lorsque l'état des plans postérieurs de la cornée du patient autorise une telle solution.

Pour atteindre ce but, l'invention fournit la cornée synthétique selon la revendication 1.

On comprend que, grâce à la forme générale en tronc de cône de la prothèse, celle-ci peut être logée dans un évidement de la partie centrale de la cornée ayant une forme similaire sans qu'il soit nécessaire de perforer la membrane de Descemet et l'endothélium. Néanmoins, grâce à la forme de tronc de cône, la comée synthétique est efficacement maintenue dans la cornée, en appui sur la membrane de Descemet. Au-delà d'un angle a égal à 35 degrés, les risques d'expulsion deviennent importants.

De préférence, l'angle a est de l'ordre de 25 à 30 degrés, ce qui assure un maintien encore meilleur de la prothèse dans la cornée.

De préférence encore, le rayon de courbure de la face antérieure de la pièce transparente est aussi proche que possible de celui de la face antérieure de la comée, c'est-à-dire compris entre 6,5 et 8,5 mm. Ainsi, on évite les risques d'une perturbation des mouvements des paupières sur la cornée, préjudiciables mécaniquement à la contention.

Un autre problème qui se pose en cas de mise en place d'une cornée synthétique dans la cornée naturelle est le risque de prolifération de cellules susceptibles de venir se développer sur la face postérieure de la cornée synthétique et de rendre progressivement opaque cette face postérieure par la formation d'une membrane rétroprothétique.

Selon un mode perfectionné de réalisation de l'invention, la cornée synthétique comprend en outre une jupe annulaire raccordée à la face latérale de la pièce formant la cornée synthétique, la face postérieure de ladite jupe étant disposée sur la même calotte sphérique que la face postérieure de la cornée synthétique proprement dite, ladite jupe ayant une épaisseur au plus égale à 100 microns.

La jupe sert de barrière contre la prolifération des cellules vers la face postérieure de la cornée synthétique proprement dite. Elle n'a aucune fonction mécanique de maintien de la cornée synthétique dans la cornée naturelle, C'est pourquoi on peut lui donner une épaisseur très réduite, inférieure à 100 microns et de préférence inférieure à 50 microns. Le module de Young utilisé étant inférieur, de préférence, à 1MPa, on comprend que la jupe n'a aucune résistance mécanique significative.

Il est possible, pour améliorer encore l'effet de barrière contre la prolifération des cellules, de prévoir que le bord de la jupe soit "carré".

D'autres caractéristiques et avantages de l'invention apparaîtront mieux à la lecture de la description qui suit de plusieurs modes de réalisation de l'invention donnés à titre d'exemples non limitatifs.

Ainsi réalisée, la description se réfère aux figures annexées sur lesquelles :
- la figure 1 déjà décrite montre en coupe transversale la structure de la cornée de l'oeil ;
- la figure 2 montre une cornée synthétique selon l'invention mise en place dans la cornée ;
- la figure 3 montre en coupe verticale un mode de réalisation préféré de la cornée synthétique ;
- la figure 4 montre une première variante de réalisation de la cornée synthétique mise en place dans la cornée de l'oeil ;
- les figures 5a et 5b montrent en demi-vue de dessus et en coupe selon la figure B-B une deuxième variante de réalisation de la cornée synthétique ; et
- la figure 6 montre en coupe verticale une troisième variante de réalisation de la cornée synthétique.

La cornée synthétique est destinée à être mise en place dans une trépanation lamellaire profonde devant la membrane de Descemet réalisée dans la partie centrale de la cornée. Sur la figure 2, on a représenté cet évidement 28 qui présente un axe de révolution YY'. L'évidement comporte une paroi tronconique 30 dont le sommet est du côté de la face antérieure de la cornée et qui s'arrête à la membrane de Descemet 16. Cet évidement a ainsi une profondeur e', il présente dans la face antérieure de la cornée un diamètre D₂' et, à proximité de la membrane de Descemet, un diamètre D'₁.

En se référant maintenant à la figure 3, on va décrire plus en détail un premier mode de réalisation de la cornée synthétique 40. Dans ce mode de réalisation, la cornée synthétique consiste en une pièce unique 42 qui présente un axe de révolution XX'. La pièce 42 est définie par une face antérieure 44 en forme de calotte sphérique de rayon Rₐ, une face postérieure 46 également en forme de calotte sphérique et de rayon Rₚ et par une paroi latérale 48 qui raccorde les faces antérieure 44 et postérieure 46 de la pièce 42. La paroi latérale 48 a la forme d'un tronc de cône dont l'axe est l'axe XX' et dont le sommet est tourné vers la face antérieure 44 de la pièce 42. La face antérieure 44 est limitée par un cercle C2 de diamètre D₂ et la face postérieure 46 est limitée par un cercle C₁ de diamètre D₁. On a représenté par a l'angle que fait, à proximité du cercle C₁, le raccordement entre la paroi latérale 48 et la face postérieure 46 en section par un plan contenant l'axe XX'.

Plus précisément, l'angle a est l'angle que fait, dans un plan de section contenant l'axe XX', la section de la paroi latérale de la prothèse avec la tangente T à la section de la face postérieure 46 au point de raccordement E.

Pour assurer un bon maintien de la cornée synthétique, l'angle a est inférieur à 35 degrés et, de préférence, de l'ordre de 25 à 30 degrés.

De préférence, le diamètre D₁ est compris entre 4,5 et 9,5 à 10 mm, le diamètre D₂ est compris entre 3 mm et 8 mm, la distance e séparant les faces antérieure et postérieure, dans leurs parties centrales, est comprise entre 400 et 500 µm.

L'épaisseur e de la prothèse dans sa partie centrale est sensiblement égale à l'épaisseur de la cornée dans sa partie centrale puisque seules demeurent la membrane de Descemet et l'endothélium qui ont des épaisseurs très réduites.

L'évidement 28 qui a été réalisé dans la cornée a une forme similaire à celle de la pièce 42, mais de dimension légèrement réduite, de telle manière qu'une pression soit exercée sur la face latérale de la cornée synthétique par le bord latéral du logement réalisé dans la cornée. Plus précisément, de préférence, les diamètres D'1 et D'2 sont inférieurs de 250 microns aux valeurs des diamètres correspondant de la pièce 42.

La pièce 42 doit être réalisée avec un matériau biocompatible souple. Ce matériau peut être un acrylique hydrophile ou hydrophobe. Les acryliques hydrophiles peuvent être choisis parmi les copolymères du méthacrylate d'hydroxyéthyle, du méthacrylate d'hydroxypropyle, du méthacrylate d'hydroxybutyle et du méthacrylate d'hydroxyhexyle.

Pour ce qui est des acryliques hydrophobes, on utilisera de préférence des composés fluorés d'acrylate ou de méthacrylate.

On peut obtenir les acryliques hydrophobes par copolymérisation d'un acrylate et d'un méthacrylate, l'acrylate ayant une température de transition vitreuse négative et le méthacrylate ayant une température de transition nettement positive (par exemple de 110° C pour le méthacrylate) de telle manière que l'acrylique soit souple à la température ambiante.

Il faut souligner que les rayons de courbures Rₐ et R_{b} des faces antérieure et postérieure de la cornée synthétique sont idéalement choisis de telle manière que ces rayons soient sensiblement égaux à ceux de la cornée, de telle manière que notamment la face antérieure de la cornée synthétique soit disposée sur la même calotte sphérique que la face antérieure de la cornée.

Cependant, cette solution optimale ne peut pas, le plus souvent, être satisfaite. D'une part il faut que la face postérieure 46 soit efficacement appliquée sur la membrane de Descemet. Pour obtenir ce résultat, il est intéressant de prévoir un rayon de courbure un peu supérieur à celui de la cornée. C'est pourquoi on choisit un rayon compris de préférence entre 6 et 8 mm.

De plus, comme on l'a expliqué précédemment, on peut utiliser différents matériaux pour réaliser la cornée synthétique, ces matériaux ayant des indices optiques différents. Par exemple, pour certains acryliques, l'indice optique est de 1,38, c'est-à-dire très proche de celui de la cornée. Il sera alors possible de donner à la face antérieure un rayon de courbure compris entre 6,5 et 8,5 mm, c'est-à-dire proche de celui de la cornée, tout en gardant à celle-ci une vergence de 40 dioptries. Avec d'autres acryliques, l'indice optique est de 1,465. Dans ce cas, il faudra donner à la face antérieure un rayon de courbure bien plus élevé pour conserver une vergence de 40 dioptries, par exemple de l'ordre de 19 à 25 mm.

On comprend que, grâce à la forme en tronc de cône du logement 28 réalisé dans la cornée et à la forme conjuguée en tronc de cône de la cornée synthétique, celle-ci est maintenue mécaniquement dans le logement dont le fond qui présente la plus grande dimension est fermé par la membrane de Descemet et par l'endothélium. Ainsi, la cornée synthétique est maintenue efficacement dans la cornée, bien que la trépanation réalisée dans celle-ci ne soit pas transfixiante. Cependant, pour garantir une bonne intégration dans la période qui suit immédiatement l'intervention chirurgicale, on ajoute de préférence de la colle, colle qui se résorbera au bout de quelques jours ou quelques semaines.

En se référant maintenant à la figure 4, on va décrire un deuxième mode de réalisation de cornée synthétique qui porte la référence 40'. Cette cornée synthétique comporte une pièce transparente 42 identique à celle qui est représentée sur la figure 3 et une corolle 60 réalisée en matériau biocolonisable. La corolle 60 est de révolution autour de l'axe XX' de la pièce 42. Elle comprend une première portion tronconique 62 qui est appliquée contre la paroi latérale tronconique 48 de la pièce 42. Elle est ancrée dans la pièce 42 par une extension 64 qui pénètre dans une gorge annulaire 66 ménagée dans la paroi latérale de la pièce 42. Cette corolle 60 se prolonge par une jupe 68 qui va en s'écartant de la pièce 42 à proximité de sa face postérieure 46. Cette jupe 68 est destinée à être insérée dans un délaminage 70 entre le stroma et la membrane 18 de Descemet 16.

La corolle 60 est réalisée en un matériau biocolonisable qui est de préférence du polychlorotrifluoroéthylène. La corolle 60 a une épaisseur au plus égale à 100 microns et, de préférence, égale à 25 microns. La partie formant jupe 68 de la corolle 60 est de préférence munie de trous de diamètre compris entre 50 et 100 microns pour améliorer l'ancrage de la corolle dans la cornée. La corolle a pour fonction de favoriser la biocolonisation après la mise en place de la cornée synthétique 40' dans la cornée.

Il est également possible dé greffer sur la face antérieure 44 de la cornée synthétique un revêtement 72 facilitant la colonisation cellulaire de la prothèse afin d'en améliorer l'intégration dans la cornée. De même, sur la face postérieure 46 de la cornée synthétique, on peut greffer un revêtement 74 pour inhiber la prolifération cellulaire et éviter ainsi la propagation d'un épithélium s'accompagnant d'une expulsion de la prothèse. Les revêtements 72 et 74 peuvent bien sûr être prévus pour la cornée synthétique 40 de la figure 3.

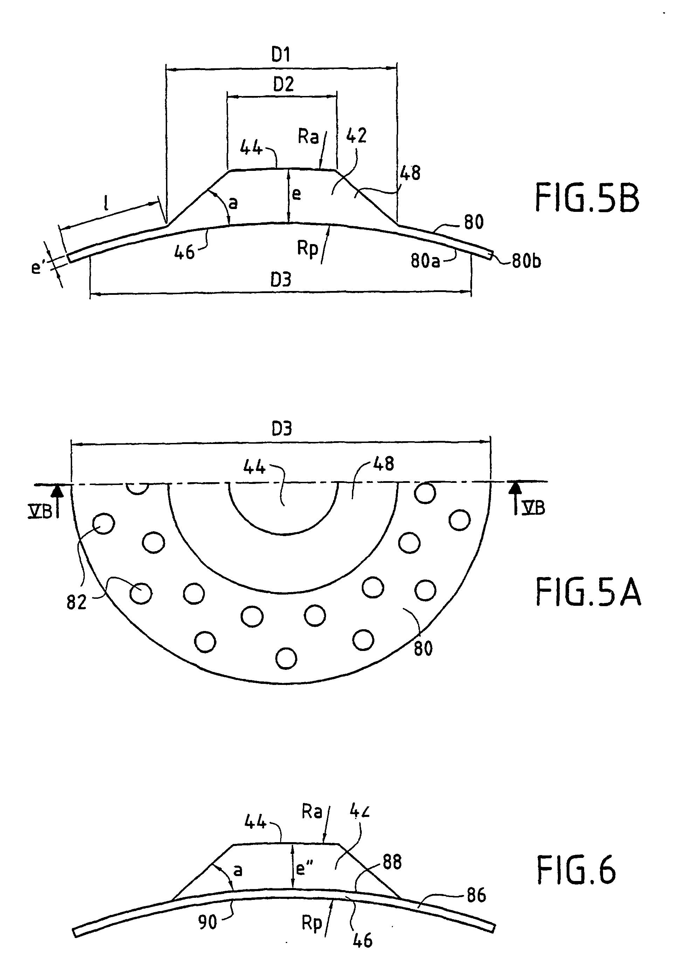

En se référant maintenant aux figures 5a et 5b, on va décrire un troisième exemple de réalisation de la cornée synthétique. Celle-ci est constituée par la pièce 42 identique à celles qui ont déjà été décrites et par une jupe annulaire 80 disposée à proximité de la face postérieure 46 de la pièce 42. Plus précisément, la face postérieure de la jupe 80 référencée 80a est disposée sur la même calotte sphérique que la face postérieure 46 de la pièce 42. La jupe 80 présente une épaisseur e' qui est comprise entre 10 et 100 µm et de préférence entre 20 et 50 µm et la largeur I de cette jupe est comprise entre 0,25 et 2,5 mm et de préférence entre 0,75 et 1,5 mm. De préférence, la jupe 80 est pourvue d'orifices tels que 82 dont le diamètre est compris entre 10 et 100 µm et de préférence entre 50 et 80 µm afin de favoriser la biocolonisation de ses trous par les kératocytes et fibrocytes cornéens et obtenir ainsi une fixation à long terme. Les trous 82 peuvent être remplis au préalable par un matériau biodégradable collant, par exemple fibrinogène capable de relarguer un agent qui favorise la biocolonisation et la formation de pont en collagène entre la membrane de Descemet de la cornée et la stroma cornéenne.

La jupe 80 peut être réalisée avec le même matériau que la partie optique ou dans un autre matériau. Le matériau constituant la jupe 80 pourrait être un chlorotrifluoroéthylène ou un tétrafluoroéthylène, alors que la pièce 42 proprement dite serait réalisée en hydrogel.

Comme on l'a déjà expliqué, la cornée synthétique est maintenue dans la cornée naturelle grâce à la forme tronconique très évasée de la pièce 42. La jupe 80 a seulement pour fonction de créer une barrière contre la prolifération des cellules vers la face postérieure de la cornée synthétique. Elle peut ainsi avoir une épaisseur réduite typiquement inférieure à 50 microns. Cette épaisseur très réduite permet de limiter la déformation de la cornée dans la zone annulaire occupée par la jupe 80.

Il est possible que le bord latéral 80b de la jupe 80 soit perpendiculaire aux faces antérieure et postérieure de la jupe. On obtient ainsi des "bords carrés" qui "bloquent" la prolifération des cellules.

La surface externe latérale 48 de la pièce 42 et la totalité de la surface de la jupe 80 sont de préférence traitées pour favoriser et accélérer la prolifération épitaxiale, alors que la face postérieure 46 de la pièce 42 sera traitée afin de prévenir une telle prolifération.

Dans le cas du mode de réalisation représenté sur la figure 6, la collerette est constituée par une pièce continue 86 dont la partie centrale antérieure 88 est soudée sur la face postérieure 86 de la pièce 42. La face postérieure 90 de la pièce 86 présente le rayon de courbure Rp correspondant au rayon de courbure de la membrane de Descemet. Dans ce cas, comme on l'a expliqué précédemment, la pièce 42 et l'ensemble formant collerette 86 peuvent être constitués par le même matériau ou par des matériaux différents. Dans ce cas encore, la partie de la pièce 86 non recouverte par la pièce 42 peut comporter avantageusement des trous identiques aux trous 82 de la figure 5a. Enfin, c'est bien sûr l'épaisseur totale de la pièce 42 et de la collerette qui devra être égale à la valeur e mentionnée précédemment.

Ce mode de réalisation présente toutes les caractéristiques et avantages du mode de réalisation des figures 5a et 5b.

Lorsque la cornée synthétique est réalisée avec un acrylique hydrophobe, celui-ci est, de préférence, choisi dans le groupe comprenant l'heptafluorobutyle acrylate, l'hexafluorobutyle acrylate et les composés fluorés ayant un indice de réfraction proche de celui de la cornée humaine.

## Revendications

1. Cornée synthétique destinée à être placée dans un évidement (28) ménagé dans la cornée de l'oeil, ledit évidement débouchant dans la face antérieure de la cornée et non dans sa face postérieure pour laisser subsister la membrane de Descemet (16), **caractérisée en ce que** la partie optique de ladite cornée consiste en une pièce unique (42) entièrement réalisée en un matériau transparent souple à axe de symétrie de révolution présentant une face antérieure (44) en forme de calotte sphérique de rayon Rₐ et une face postérieure (46) en forme de calotte sphérique de rayon Rₚ, les deux faces étant séparées, dans leurs zones centrales, par une distance e et une paroi latérale (48) en forme sensiblement de tronc de cône d'axe confondu avec l'axe de révolution et dont le sommet est disposé du côté de ladite face antérieure, de telle manière que la section perpendiculaire à l'axe de symétrie croît continûment de D2 à D1, l'angle a que fait la section de la paroi latérale et de la face postérieure par un plan contenant ledit axe de révolution étant compris entre 10 et 35 degrés.

2. Cornée synthétique selon la revendication 1, **caractérisée en ce que** ledit angle a est de l'ordre de 25 à 30 degrés.

3. Cornée synthétique selon l'une quelconque des revendications 1 et 2, **caractérisée en ce que** la distance e de ladite pièce est comprise entre 400 et 500 microns.

4. Cornée synthétique selon l'une quelconque des revendications 1 à 3, **caractérisée en ce que** le diamètre du cercle limitant la face antérieure (44) de la pièce est compris entre 3 et 8 mm et **en ce que** le diamètre du cercle limitant la face postérieure (46) de la pièce est compris entre 4,5 et 9,5 mm.

5. Cornée synthétique selon l'une quelconque des revendications 1 à 4, **caractérisée en ce que** le rayon de courbure Rₚ de la face postérieure est compris entre 6 et 8 mm.

6. Cornée synthétique selon l'une quelconque des revendications 1 à 5, **caractérisée en ce que** le rayon de courbure Rₐ de ladite face antérieure est compris entre 6,5 et 8,5 mm.

7. Cornée synthétique selon l'une quelconque des revendications 1 à 6, **caractérisée en ce que** ledit matériau constituant ladite pièce est un acrylique hydrophile.

8. Cornée synthétique selon la revendication 7, **caractérisée en ce que** ledit acrylique hydrophile est choisi dans le groupe comprenant les copolymères du méthacrylate d'hydroxyéthyle, du méthacrylate d'hydroxypropyle, du méthacrylate d'hydroxybutyle et du méthacrylate d'hydroxyhexyle.

9. Cornée synthétique selon l'une quelconque des revendications 1 à 6, **caractérisée en ce que** ledit matériau dont est faite ladite pièce est un acrylique hydrophobe.

10. Cornée synthétique selon la revendication 9, **caractérisée en ce que** ledit acrylique hydrophobe est choisi dans le groupe comprenant l'heptafluorobutyle acrylate, l'hexafluorobutyle acrylate et les composés fluorés ayant un indice de réfraction proche de celui de la cornée humaine.

11. Cornée synthétique selon l'une quelconque des revendications 1 à 10, **caractérisée en ce que** la face antérieure (44) de ladite pièce est revêtue d'un matériau (72) favorisant la prolifération cellulaire.

12. Cornée synthétique selon l'une quelconque des revendications 1 à 11, **caractérisée en ce que** la face postérieure (46) de ladite pièce est revêtue d'un matériau inhibant la prolifération cellulaire.

13. Cornée synthétique selon l'une quelconque des revendications 1 à 12, **caractérisée en ce qu'**elle comprend en outre une corolle (60) de révolution réalisée en un matériau biocolonisable dont une extrémité est solidaire de la paroi latérale (48) de ladite pièce (42) et dont l'autre extrémité (68) est libre.

14. Cornée synthétique selon la revendication 13, **caractérisée en ce que** ladite corolle est réalisée en polychlorotrifluoroéthylène.

15. Cornée synthétique selon l'une quelconque des revendications 1 à 12, **caractérisée en ce qu'**elle comprend en outre une jupe annulaire (80, 86) raccordée à la face latérale de ladite pièce (42), la face postérieure (80a, 90) de ladite jupe étant disposée sur la même calotte sphérique que la face postérieure de ladite pièce (42), l'épaisseur (e) de ladite jupe étant au plus égale à 100 microns.

16. Cornée synthétique, selon la revendication 15, **caractérisée en ce que** ladite jupe est réalisée avec le même matériau que ladite pièce.

17. Cornée synthétique, selon la revendication 15, **caractérisée en ce que** ladite jupe est réalisée avec un matériau différent de celui de ladite pièce.

18. Cornée synthétique selon l'une quelconque des revendications 15 à 17, **caractérisée en ce que** l'épaisseur de ladite jupe est comprise entre 20 et 50 µm.

## Patentansprüche

1. Künstliche Hornhaut, die dazu bestimmt ist, in eine Aussparung (28) eingesetzt zu werden, die in der Hornhaut des Auges angeordnet ist, wobei die Aussparung in die vordere Fläche der Hornhaut mündet und nicht in ihre rückwärtige Fläche, um die Descemet-Membran (16) bestehen zu lassen, **dadurch gekennzeichnet, daß** der optische Teil der Hornhaut aus einem einzigen Stück (42) besteht, das vollkommen aus einem durchsichtigen weichen Material mit einer rotationssymmetrischen Achse ausgeführt ist, das eine vordere Fläche (44) in Form einer kugelförmigen Kappe mit einem Radius Rₐ und eine rückwärtige Fläche (46) in Form einer kugelförmigen Kappe mit einem Radius Rₚ aufweist, wobei die beiden Flächen in ihren Mittelbereichen voneinander getrennt sind durch einen Abstand e und eine seitliche Wand (48) in einer im wesentlichen kegelstumpfförmigen Form mit einer Achse, die mit der Rotationsachse verschmolzen ist, und deren Scheitelpunkt auf der Seite der vorderen Fläche so angeordnet ist, daß der Abschnitt, der zur Symmetrieachse senkrecht ist, kontinuierlich von D2 nach D1 zunimmt, wobei der Winkel a, den der Abschnitt der seitlichen Wand und die vordere Fläche durch eine Ebene ausbilden, welche die Rotationsachse enthält, zwischen 10 und 35 Grad liegt.

2. Künstliche Hornhaut nach Anspruch 1, **dadurch gekennzeichnet, daß** der Winkel a eine Größenordnung von 25 bis 30 Grad aufweist.

3. Künstliche Hornhaut nach einem der Ansprüche 1 und 2, **dadurch gekennzeichnet, daß** der Abstand e des Stücks zwischen 400 und 500 Mikron beträgt.

4. Künstliche Hornhaut nach einem der Ansprüche 1 bis 3, **dadurch gekennzeichnet, daß** der Durchmesser des Kreises, der die vordere Fläche (44) des Stücks begrenzt, zwischen 3 und 8 mm beträgt, und dadurch, daß der Durchmesser des Kreises, der die rückwärtige Fläche (46) begrenzt, zwischen 4,5 und 9,5 mm beträgt.

5. Künstliche Hornhaut nach einem der Ansprüche 1 bis 4, **dadurch gekennzeichnet, daß** der Krümmungsradius Rₚ der rückwärtigen Fläche zwischen 6 und 8 mm beträgt.

6. Künstliche Hornhaut nach einem der Ansprüche 1 bis 5, **dadurch gekennzeichnet, daß** der Krümmungsradius Rₐ der vorderen Fläche zwischen 6,5 und 8,5 mm beträgt.

7. Künstliche Hornhaut nach einem der Ansprüche 1 bis 6, **dadurch gekennzeichnet, daß** das Material, aus dem das Stück ausgebildet ist, ein hydrophiles Acryl ist.

8. Künstliche Hornhaut nach Anspruch 7, **dadurch gekennzeichnet, daß** das hydrophile Acryl aus der Gruppe ausgewählt wird, welche die Copolymere des Hydroxyethylmethacrylats, Hydroxypropylmethacrylats, Hydroxybutylmethacrylats und Hydroxyhexylmethacrylats enthält.

9. Künstliche Hornhaut nach einem der Ansprüche 1 bis 6, **dadurch gekennzeichnet, daß** das Material, aus dem das Stück ausgebildet ist, ein hydrophobes Acryl ist.

10. Künstliche Hornhaut nach Anspruch 9, **dadurch gekennzeichnet, daß** das hydrophile Acryl aus der Gruppe ausgewählt wird, welche das Heptafluorbutylacrylat, das Hexafluorbutylacrylat und die fluorierten Verbindungen mit einem Brechungsindex enthält, der demjenigen der menschlichen Hornhaut nahe ist.

11. Künstliche Hornhaut nach einem der Ansprüche 1 bis 10, **dadurch gekennzeichnet, daß** die vordere Fläche (44) des Stücks mit einem Material (72) verkleidet ist, das die Zellvermehrung begünstigt.

12. Künstliche Hornhaut nach einem der Ansprüche 1 bis 11, **dadurch gekennzeichnet, daß** die rückwärtige Fläche (46) des Stücks mit einem Material verkleidet ist, das die Zellvermehrung verhindert.

13. Künstliche Hornhaut nach einem der Ansprüche 1 bis 12, **dadurch gekennzeichnet, daß** sie des weiteren eine Drehkrone (60) aufweist, die aus einem sich biologisch verwachsenden Material ausgeführt ist, von der ein Ende mit der seitlichen Wand (48) des Stücks (42) fest verbunden ist und deren anderes Ende (68) frei ist.

14. Künstliche Hornhaut nach Anspruch 13, **dadurch gekennzeichnet, daß** die Krone aus Polychlortrifluorethylen ausgeführt ist.

15. Künstliche Hornhaut nach einem der Ansprüche 1 bis 12, **dadurch gekennzeichnet, daß** sie des weiteren eine ringförmige Schürze (80, 86) umfaßt, die mit der seitlichen Fläche des Stücks (42) verbunden ist, wobei die rückwärtige Fläche (80a, 90) der Schürze auf der gleichen kugelförmigen Kappe angeordnet ist wie die rückwärtige Fläche des Stücks (42), wobei die Dicke (e) der Schürze höchstens gleich 100 Mikron ist.

16. Künstliche Hornhaut nach Anspruch 15, **dadurch gekennzeichnet, daß** die Schürze aus dem gleichen Material ausgeführt ist wie das Stück.

17. Künstliche Hornhaut nach Anspruch 15, **dadurch gekennzeichnet, daß** die Schürze aus einem anderen Material als demjenigen des Stücks ausgeführt ist.

18. Künstliche Hornhaut nach einem der Ansprüche 15 bis 17, **dadurch gekennzeichnet, daß** die Dicke der Schürze zwischen 20 und 50 µm beträgt.

## Claims

1. A synthetic cornea for placing in a recess (281) formed in the cornea of the eye, said recess opening out into the anterior face of the cornea and not into its posterior face so as to leave Descemet's membrane (16) intact, the synthetic cornea being **characterised in that** the optical portion of said cornea consists of a single piece (42) made entirely of flexible transparent material having an axis of circular symmetry presenting an anterior face (44) in the form of a spherical cap of radius Rₐ and a posterior face (46) in the form of a spherical cap of radius Rₚ, the two faces being spaced apart in their central zones by a distance e, and a side wall (48) substantially in the form of a truncated cone having its axis coinciding with the axis of symmetry and having its apex disposed in front of said anterior face, in such a manner that its section that is perpendicular to the axis of symmetry increases continuously from D2 to D1, the angle a between the section of the side wall and the section of the posterior face in a section plane containing said axis of symmetry lying in the range 10° to 35°.

2. A synthetic cornea according to claim 1, **characterised in that** said angle a is about 25° to 30°.

3. A synthetic cornea according to claim 1 or claim 2, **characterised in that** the distance e in said piece lies in the range 400 µm to 500 µm.

4. A synthetic cornea according to any one of claims 1 to 3, **characterised in that** the diameter of the circle defining the anterior face (44) of the piece lies in the range 3 mm to 8 mm, and **in that** the diameter of the circle defining the posterior face (46) of the piece lies in the range 4.5 mm to 9.5 mm.

5. A synthetic cornea according to any one of claims 1 to 4, **characterised in that** the radius of curvature Rₚ of the posterior face lies in the range 6 mm to 8 mm.

6. A synthetic cornea according to any one of claims 1 to 5, **characterised in that** the radius of curvature Rₐ of said anterior face lies in the range 6.5 mm to 8.5 mm.

7. A synthetic cornea according to any one of claims 1 to 6, **characterised in that** said material constituting said piece is a hydrophilic acrylic.

8. A synthetic cornea according to claim 7, **characterised in that** said hydrophilic acrylic is selected from the group comprising copolymers of hydroxyethyl methacrylate, of hydroxypropyl methacrylate, of hydroxybutyl methacrylate, and of hydroxyhexyl methacrylate.

9. A synthetic cornea according to any one of claims 1 to 6, **characterised in that** said material from which said piece is made is a hydrophobic acrylic.

10. A synthetic cornea according to claim 9, **characterised in that** said hydrophobic acrylic is selected from the group comprising heptafluorobutyl acrylate, hexafluorobutyl acrylate, and fluorine-containing compounds having a refractive index close to that of the human cornea.

11. A synthetic cornea according to any one of claims 1 to 10, **characterised in that** the anterior face (44) of said piece is coated in a material (72) that encourages cellular proliferation.

12. A synthetic cornea according to any one of claims 1 to 11, **characterised in that** the posterior face (46) of said piece is coated in a material that inhibits cellular proliferation.

13. A synthetic cornea according to any one of claims 1 to 12, **characterised in that** it further comprises a circular ring (60) made of a biocolonizable material having one end secured to the side wall (48) of said piece (42) and having its other end (68) free.

14. A synthetic cornea according to claim 13, **characterised in that** said ring is made of polychlorotrifluoroethylene.

15. A synthetic cornea according to any one of claims 1 to 12, **characterised in that** it further comprises an annular skirt (80, 86) connected to the side face of said piece (42), the posterior face (80a, 90) of said skirt being disposed on the same spherical cap as the posterior face of said piece (42), the thickness (e) of said skirt being no greater than 100 µm.

16. A synthetic cornea according to claim 15, **characterised in that** said skirt is made out of the same material as said piece.

17. A synthetic cornea according to claim 15, **characterised in that** said skirt is made of a material different from that of said piece.

18. A synthetic cornea according to any one of claims 15 to 17, **characterised in that** the thickness of said skirt lies in the range 20 µm to 50 µm.
